# EUROPÄISCHE PATENTANMELDUNG

(11) **EP 0 891 785 A2**
(43) Veröffentlichungstag der Anmeldung: **20.01.1999**
(21) Anmeldenummer: 98108217.5
(22) Anmeldetag: 06.05.1998
(51) Int. Cl.: A61M 5/32

(54) **Kanülenbehälter**

(30) Priorität: 16.07.1997 DE 19730472
(71) Anmelder: Rigling, Heinz, D-75382 Althengstett (DE)
(72) Erfinder: Rigling, Heinz, D-75382 Althengstett (DE)
(74) Vertreter: Nix, Frank Arnold, Dr.

(57) **Zusammenfassung**

Im Deckel 2 des Behälters für gebrauchte Kanülen ist wenigstens eine Abwerföffnung 4 ausgebildet, die mittels eines Verschlusses 13 verschließbar ist, in welchem mit einem Abstand 17 wenigstens eine Ventilationsöffnung 20 derart versetzt ausgeführt ist, daß die Kanülen keine Möglichkeit des Austritts nach außen haben. Die Ventilationsöffnungen 20 des Verschlusses sind von einer Schmelzplatte 30 überdeckt, die bei den während der Sterilisation im Autoklaven auftretenden Temperaturen schmilzt.

## Beschreibung

Die vorliegende Erfindung bezieht sich auf einen Behälter für gebrauchte Kanülen, meist Kanülenbox genannt, wie er in Arztpraxen und Krankenhäusern benutzt wird, um für Injektionen, Infusionen oder dergleichen verwendete Nadeln und Kanülen bis zur Entsorgung aufzubewahren. Die Entsorgung von infektiösem Abfall ("C-Müll") ist aufwendig und nur in speziellen Einrichtungen möglich.

Wenn die endgültige Entsorgung eines Kanülenbehälters mit Inhalt als nicht kontaminierter Abfall ("A-Müll") ohne besondere Maßnahmen geschehen soll, beispielsweise durch allgemeines Verbrennen in einer Hausmüll-Verbrennungsanlage oder durch Lagern auf einer Hausmülldeponie, so muß vorher eine Sterilisation stattfinden. Eine solche geschieht in Autoklaven, in die zunächst ein Unterdruck eingeleitet wird, um das Behälterinnere zu evakuieren, und danach überhitzter Dampf eingeleitet wird, der dann den gesamten Behälterinnenraum füllt und eine zuverlässige Sterilisation der gebrauchten Kanülen gewährleistet. Dieser Vorgang wird gewöhnlich mehrere Male wiederholt.

Ein aus DE-GM 93 18 733 bekannter Kanülenbehälter besteht aus einem Behälterkörper und einem Deckel, in dem wenigstens eine Abwerföffnung ausgebildet ist, deren Umriß eine weitere und eine engere Stelle bildet. Zum Abwerfen einer Kanüle wird die Spritze mit aufgesetzter Kanüle so in den Behälter getaucht, daß der Abschnitt zwischen Stirnfläche der Spritze und dem am hinteren Ende der Kanüle befindlichen Kanülenflansch sich in Höhe der Abwerföffnung befindet, worauf die Spritze seitlich in die enge Stelle der Abwerföffnung bewegt wird, sodaß der Kanülenflansch sich unterhalb derselben befindet. Dann wird die Spritze zurückgezogen, wobei der Kanülenflansch sich an der Unterfläche des Deckels beidseits der Verengung der Abwerföffnung anlegt und die Kanüle von der Spritze abgezogen wird und ins Behälterinnere fällt.

Wenn der Behälter voll ist, wird die Abwerföffnung verschlossen und der Behälter in diesem Zustand der Sterilisation und der endgültigen Entsorgung zugeführt. Dies macht es nötig, den Verschluß vor der Sterilisation wieder zu entfernen und danach wieder anzubringen, was aufwendig und wegen der Möglichkeit des Austretens von Kanülen gefährlich ist.

Von einem solchen Kanülenbehälter aus einem Behälterkörper und einem Deckel, in dem wenigstens eine mittels eines flächigen Verschlusses verschließbare Abwerföffnung ausgebildet ist, geht die vorliegende Erfindung aus.

Aufgabe der Erfindung ist die Schaffung eines Kanülenbehälters, der nach dem Verschließen der Abwerföffnung ohne nochmaliges Öffnen sterilisiert und der endgültigen Entsorgung zugeführt werden kann.

Die Lösung der gestellten Aufgabe gelingt durch die im Patentanspruch 1 angegebenen Merkmale. Zweckmäßige Weiterbildungen sind in den Unteransprüchen angegeben. Dabei zielt speziell Anspruch 3 auf eine Ausbildung, die es erlaubt, den Behälter nach Befüllung dicht zu verschließen, sodaß die Aufbewahrung und der Transport zum Autoklaven keine Probleme aufwirft.

Die Erfindung wird nachfolgend durch die Beschreibung von zwei Ausführungsbeispielen anhand der beigegebenen Zeichnungen weiter erläutert. Es zeigt:
- Fig. 1: einen teilweisen Längsschnitt durch einen Kanülenbehälter einer ersten Ausbildungsform;
- Fig. 2: einen teilweisen Längsschnitt durch einen Kanülenbehälter einer zweiten Ausbildungsform.

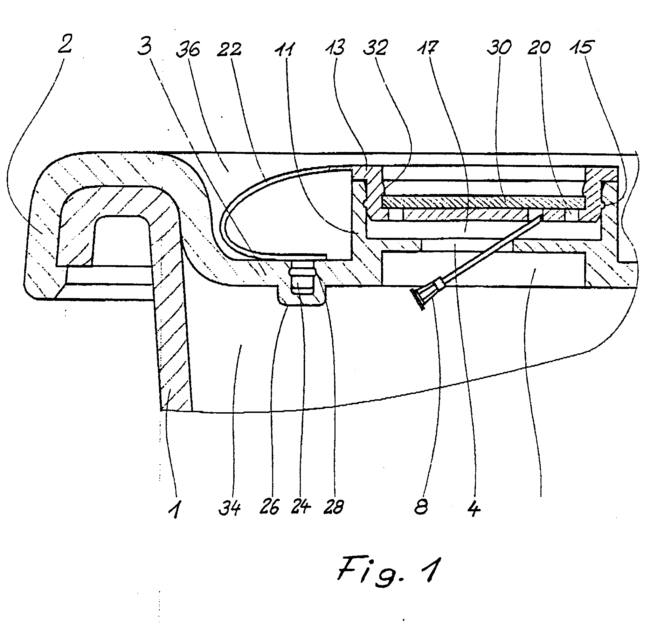

Der aus Fig. 1 ersichtliche Kanülenbehälter besteht aus einem Behälterkörper 1, auf dessen oberen Rand ein Deckel 2 so aufgesetzt ist, daß er sich bei normalen Handhabungen nicht lösen kann. Hierzu ist der obere Rand des Behälterkörpers nach außen und unten abgebogen und die Stirnfläche dieser Abbiegung nach unten wird in der aus der Zeichnung ersichtlichen Weise hintergriffen von einem entsprechend geformten Rand des Deckels 2.

In der Fläche 3 des Deckels ist eine Abwerföffnung 4 ausgebildet, deren Rand Einbuchtungen aufweist, deren Breite kleiner ist als der Durchmesser des am hinteren Ende der Kanülen 8 befindlichen Flanschs 9. Zum Abwerfen einer Kanüle 8 wird die Spritze mit aufgesteckter Kanüle so in den Behälter versenkt, daß der Flansch unterhalb einer Einbuchtung zu liegen kommt und beim Hochziehen der Spritze die Kanüle von der Spritze abgezogen wird und ins Behälterinnere fällt.

Die Abwerföffnung 4 ist umgeben von einem aufragenden Kragen 11, der mittels eines Verschlusses 13 überdeckbar ist. In der gezeigten Ausbildung ist ein tellerartiger Verschluß 13 in das obere Ende des Kragens 11 einsetzbar. Eine andere Möglichkeit wäre eine kappenartige Ausbildung, die den Kragen 11 übergreift.

In der zylindrischen Umfangsfläche des Verschlußtellers 13 ist ein Wulst 15 ausgeformt, der in eine entsprechende umlaufende Ausnehmung der Innenfläche des Kragens 11 greift und den Sitz sichert. Zwischen der Fläche des Verschlusses 13 und der überdeckten Deckelfläche mit der Abwerföffnung 4 verbleibt ein Abstand 17.

In der Verschlußfläche sind Ventilationsöffnungen 20 ausgespart, die gegenüber der Abwerföffnung 4 oder den eventuell mehreren Abwerföffnungen so versetzt sind, daß eine Kanüle 20 keine Möglichkeit hat, nach außen zu gelangen, wie dies aus der Zeichnung ersichtlich ist. Diese Bedingung ist erfüllt, wenn es in keiner Schnittebene die Möglichkeit gibt, eine Gerade durch sowohl eine Ventilationsöffnung als auch eine Abwerföffnung zu legen.

Der Verschluß 13 ist über einen biegsamen Steg 22 mit dem Deckel 6 verbunden. In der betrachteten Ausführung ist der Steg 22 am Verschluß 13 angeformt und hat an seinem Ende einen Pfropfen 24, der in eine in der Deckelfläche gebildete Ankertasche 26 gesteckt ist. Auf diese Weise kann der Verschluß 13 nicht verloren gehen, wenn er während des Füllens des Behälters mit Kanülen nicht aufgesetzt ist. Ein Wulst 28 des Pfropfens sichert den Sitz in Zusammenwirkung mit einer dem Wulst entsprechenden umlaufenden Ausnehmung der Ankertasche 26.

Zur Überdeckung der Ventilationsöffnungen 20 ist in den Verschlußteller 13 auf dessen Fläche eine Schmelzplatte 30 eingesetzt. Diese ist an ihrem Rand durch einen im Schnitt nasenförmigen, nach innen ragenden Vorsprung 32 gehalten.

Die Gestaltung des Randes des Deckels 2 ist derart, daß die Deckelfläche 3 in den Innenraum 34 des Behälters so weit versetzt ist, daß der Kragen 11 mit dem Verschluß 13 innerhalb der auf diese Weise gebildeten Vertiefung 36 bleibt und nicht hervorragt.

Im Gebrauch füllt sich der Behälter auf die übliche Weise mit gebrauchten Kanülen. Wenn er dann entsorgt werden soll, wird der Verschluß 13 angebracht; vorliegend wird also der Verschlußteller in den Kragen 11 gedrückt, sodaß der Behälterinhalt dicht abgeschlossen ist. In diesem Zustand wird der Behälter in den Autoklaven gebracht.

Unter der Wirkung der Temperatur im Autoklaven schmilzt die Schmelzplatte 30, sodaß die Ventilationsöffnungen 20 freigelegt werden. Dann wird Unterdruck eingeleitet, sodaß der Behälterinnenraum 34 evalkuiert wird und der danach eingeleitete überhitzte Dampf alle Hohlräume erreicht und den Behälterinnenraum vollständig füllt, sodaß der gesamte Inhalt sterilisiert wird. Die Evakuierung und Dampfeinleitung wird gewöhnlich dreimal aufeinanderfolgend durchgeführt.

Möglich ist auch eine Ausbildung in der Weise, daß der Verschluß 13 verhältnismäßig fest im Kragen 11 gehalten ist, während die Platte 30 nur schwach im Verschluß 13 verankert ist. Dann kann die Platte 30 schon abspringen auf Grund des Druckgefälles von innen nach außen, welches beim Einleiten von Unterdruck in den Autoklaven auftritt. Die Platte 30 muß dann keine Schmelzplatte aus einem niedrigschmelzenden Werkstoff sein.

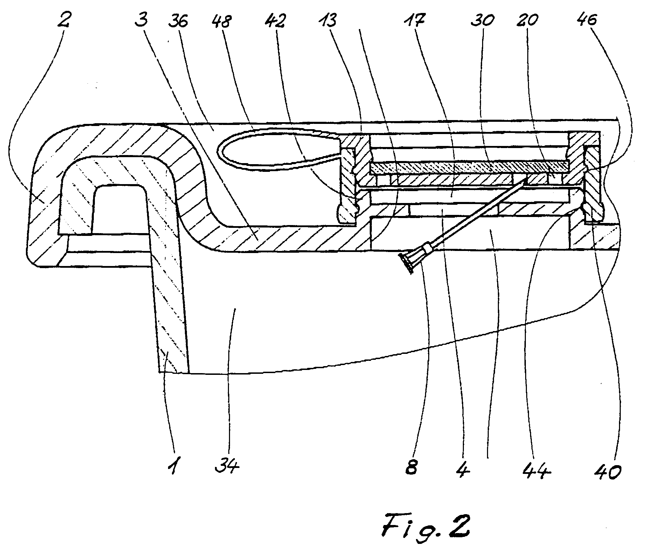

Die Ausbildung gemäß Fig. 2 unterscheidet sich nicht prinzipiell von der vorstehend betrachteten Ausbildung. Der Unterschied liegt darin, daß der Verschluß 13 nicht in einen einstückig mit dem Deckel 2 ausgebildeten Kragen einsetzbar ist, sondern in eine zylindrische Muffe 40, die ihrerseits auf einen kurzen Kragen 42 des Deckels 2 aufgesetzt ist. Zur Sicherung des Sitzes der Muffe 40 auf dem Kragen 42 dient ein umlaufender Wulst 44 und zur Sicherung des Sitzes des Verschlusses 13 in der Muffe 40 dient ein Wulst 46. Ein dünner Steg 48 verbindet den Verschluß 13 mit der Muffe 40. An der Handhabung ändert sich nichts.

### Bezugszeichenliste

- 1: Behälterkörper
- 2: Deckel
- 3: Deckelfläche
- 4: Abwerföffnung
- 8: Kanüle
- 9: Kanülenflansch
- 11: Kragen
- 13: Verschluß
- 15: Wulst
- 17: Abstand
- 20: Ventilationsöffnungen
- 22: Steg
- 24: Pfropfen
- 26: Ankertasche
- 28: Wulst
- 30: Schmelzplatte
- 32: Nasenvorsprung
- 34: Behälterinnenraum
- 36: Vertiefung
- 40: Muffe
- 42: Kragen
- 44: Wulst
- 46: Wulst
- 48: Verbindungssteg

## Patentansprüche

1. Kanülenbehälter aus einem Behälterkörper (1) und einem Deckel (2), in dem wenigstens eine mittels eines flächigen Verschlusses (13) verschließbare Abwerföffnung (4) ausgebildet ist,
dadurch gekennzeichnet, daß die Verschlußfläche in einem Abstand (17) von der die Abwerföffnung (4) enthaltenden Deckelfläche liegt
und in der Verschlußfläche Ventilationsöffnungen (20) ausgeführt sind, deren jede derart gegenüber der Abwerföffnung bzw. den Abwerföffnungen (4) versetzt ist, daß keine Kanüle (8) die Möglichkeit des Austritts aus dem Behälterinnenraum (34) hat.

2. Kanülenbehälter nach Anspruch 1, dadurch gekennzeichnet, daß der Verschluß (13) tellerartig ausgebildet ist und in einen von der Deckelfläche (3) aufragenden zylindrischen Fortsatz (11; 40) einsetzbar ist.

3. Kanülenbehälter nach Anspruch 1 oder 2, dadurch gekennzeichnet, daß die Ventilationsöffnungen (20) durch eine Schmelzplatte (30) abgedeckt sind, die aus einem Werkstoff besteht, der bei den im Autoklaven auftretenden Temperaturen schmilzt.

4. Kanülenbehälter nach Anspruch 2 und 3, dadurch gekennzeichnet, daß die Schmelzplatte (30) in der nach außen offenen Vertiefung des Verschlusses (13) mittels eines umlaufenden Nasenvorsprungs (32) gehalten ist.

5. Kanülenbehälter nach einem oder mehreren der vorhergehenden Ansprüche, gekennzeichnet durch einen den Verschluß (13) am Deckel 2 haltenden biegamen Steg (22).

6. Kanülenbehälter nach einem oder mehreren der Ansprüche 2 bis 5, dadurch gekennzeichnet, daß der zylindrische Fortsatz ein mit dem Deckel (2) einstückig ausgebildeter Kragen (11) ist.

7. Kanülenbehälter nach einem oder mehreren der Ansprüche 2 bis 5, dadurch gekennzeichnet, daß der zylindrische Fortsatz eine Muffe (40) ist, deren unterer Bereich auf einen kurzen, aus der Deckelfläche (3) aufragenden Kragen (42) aufgesetzt ist.
